# EUROPEAN PATENT SPECIFICATION

(11) **EP 2 177 602 B1**
(45) Date of publication and mention of the grant of the patent: **11.09.2013**
(21) Application number: 08703142.3
(22) Date of filing: 11.01.2008
(51) Int. Cl.: C12N 5/00

(54) **METHOD FOR ISOLATION OF CELL, SERUM-FREE CULTURE MEDIUM FOR CELL, AND METHOD FOR CULTURE OF CELL**
VERFAHREN ZUR ZELLISOLIERUNG, SERUMFREIES ZELLKULTURMEDIUM UND ZELLKULTURVERFAHREN
PROCÉDÉ D'ISOLATION DE CELLULE, MILIEU DE CULTURE EXEMPT DE SÉRUM DESTINÈ À UNE CELLULE, ET PROCÉDÉ DE CULTURE DE CELLULE

(30) Priority: 13.07.2007 JP 2007184897
(43) Date of publication of application: 21.04.2010
(73) Proprietor: Mitsubishi Tanabe Pharma Corporation, Osaka 541-8505 (JP); Yokoo, Seiichi, Tokyo 116-0012 (JP); Yamagami, Satoru, Tokyo 113-0023 (JP)
(72) Inventor: YOKOO, Seiichi, Tokyo 116-0012 (JP); YAMAGAMI, Satoru, Tokyo 113-0023 (JP)
(74) Representative: HOFFMANN EITLE
(86) International application number: PCT/JP2008/050278
(87) International publication number: WO 2009/011139

(56) References cited:
- WO-A1-2005/059113
- WO-A2-2006/044204
- WO-A2-2007/130060
- MENG ET AL: "Enhanced neural differentiation of neural stem cells and neurite growth by amniotic epithelial cell co-culture" CELL BIOLOGY INTERNATIONAL, ACADEMIC PRESS, GB LNKD- DOI:10.1016/J.CELLBI.2006.11.038, vol. 31, no. 7, 22 May 2007 (2007-05-22), pages 691-698, XP022084026 ISSN: 1065-6995
- BREWER G J ET AL: "OPTIMIZED SURVIVAL OF HIPPOCAMPAL NEURONS IN B27-SUPPLEMENTED NEUROBASAL, A NEW SERUM-FREE MEDIUM COMBINATION" JOURNAL OF NEUROSCIENCE RESEARCH, WILEY-LISS, US LNKD- DOI:10.1002/JNR.490350513, vol. 35, no. 5, 1 August 1993 (1993-08-01) , pages 567-576, XP001097842 ISSN: 0360-4012
- YOKOO SEIICHI ET AL: "Human corneal endothelial cell precursors isolated by sphere-forming assay" IOVS, vol. 46, no. 5, May 2005 (2005-05), pages 1626-1631, XP002489813 ISSN: 0146-0404
- SHIMAZAKI ET AL: "Factors Influencing Outcomes in Cultivated Limbal Epithelial Transplantation for Chronic Cicatricial Ocular Surface Disorders" AMERICAN JOURNAL OF OPHTHALMOLOGY, OPHTHALMIC PUBL, CHICAGO, IL, US LNKD- DOI:10.1016/J.AJO.2007.03.005, vol. 143, no. 6, 22 May 2007 (2007-05-22), pages 945-953, XP022088690 ISSN: 0002-9394
- NAKAMURA T ET AL: "Transplantation of Autologous Serum-Derived Cultivated Corneal Epithelial Equivalents for the Treatment of Severe Ocular Surface Disease" OPHTHALMOLOGY, J. B. LIPPINCOTT CO., PHILADELPHIA, PA, US LNKD- DOI:10.1016/J.OPHTHA.2006.04.030, vol. 113, no. 10, 1 October 2006 (2006-10-01), pages 1765-1772, XP025037691 ISSN: 0161-6420 [retrieved on 2006-10-01]
- YOKOO SEIICHI ET AL: "Human corneal epithelial equivalents for ocular surface reconstruction in a complete serum-free culture system without unknown factors" IOVS, vol. 49, no. 6, June 2008 (2008-06), pages 2438-2443, XP9135442 ISSN: 0146-0404
- YOKOO SEIICHI ET AL: "A novel isolation technique of progenitor cells in human corneal epithelium using non-tissue culture dishes" STEM CELLS (MIAMISBURG), vol. 26, no. 7, 2008, pages 1743-1748, XP9135445 ISSN: 1066-5099
- 'Adult stem cells isolated from human corneas for clinical application' CELL TECHNOLOGY vol. 26, no. 5, 22 April 2007, pages 532 - 537
- YIQIN L.L.: 'Reconstruction of chemcially burned rat corneal surface by bonw marrow-derived human mesenchymal stem cells' STEM CELLS vol. 24, no. 2, February 2006, pages 315 - 321, XP008128384
- ROUGER K. ET AL.: 'Progenitor cell isolation from muscle-derived cells based onadhesion properties' THE JOURNAL OF HISTOCHEMISTRY AND CYTOCHEMISTRY vol. 55, no. 6, June 2007, pages 607 - 618, XP008128385

## Description

### FIELD OF INVENTION

The present invention relates to a serum-free medium enabling to culture the cells under a condition in which unknown factors of animal origin and extracellular matrices are absent; and a method for culturing the cells using said serum-free medium.

### BACKGROUND ART

Stem cells are cells capable of forming organs and tissues and are thought to exist in most organs and tissues even in an adult. Among the stem cells, embryonic stem cells (ES cells) are pluripotent cells and have an ability to differentiate into any tissues and organs. On the other hand, somatic stem cells are not necessarily able to differentiate into any organs and tissues but rather differentiate into a specific tissue or organ. The somatic stem cells obtainable from human tissues can be collected from patients themselves and are less likely to cause a rejection reaction, and thus they have drawn much attention as a graft material for regenerative medicine.

As for the somatic stem cells in human, mesenchymal stem cells, hematopoietic stem cells, neural stem cells, cardiac muscle stem cells, pancreatic stem cells, skin stem cells, myeloid stem cells, retina stem cells, corneal endothelium stem cells and the like are thus far known. However, the percentage that the stem cells are contained in the tissue is extremely low, and the stem cells must be precisely separated. For separating the stem cells, a method in which proteins expressed on the cell surface which is considered to be a stem cell marker are preliminarily identified, an antibody for those proteins is prepared, and allow the antibody to bind to the marker for separation; or the like is carried out. In this method, the stem cell markers need to be identified before the separation and identification of the stem cells, and therefore reliability was low and the stem cells were not necessarily able to be precisely separated even when lots of stem cell marker candidates were used.
Furthermore, in order to prove the separated cell to be a stem cell, an ability to differentiate into other cells and an ability to form the original tissue must be strictly examined. Due to this, a serum-free medium is used in many cases for the separation and identification of the stem cells but properties of stem cells which are/have not isolated are unknown. Thus, it is very difficult to determine which factors are effective in serum-free culturing of the stem cells.

With regard to the medium used for the culturing, since factors required by the stem cells are unknown, unknown components of animal origin, such as animal serum, pituitary extracts, and released substances by co-culturing with 3T3 fibroblasts, are used for culturing most stem cells. Various factors are contained in the animal serum or pituitary extracts, there is a difference in lots between individuals, and a problem arises in confirmation of the reproducibility in examinations and/or research, or accuracy of the results. Moreover, the components of animal origin contain, in addition to the unknown factors, very enormous types of proteins, elements, lipids, vitamins and the like, and thus it is very difficult to search for a factor effective for culturing the stem cells out of the enormous types of compositions contained in the components of animal origin.

Moreover, possibilities of contamination of the cells by the known and unknown components of animal origin and pathogens in the use of the serum or pituitary extracts have been pointed out, and a solution for the problem has been desired.

Therefore, in order to solve the problems above, serum free media have been developed. For instance, in Japanese Laid-open Patent Application (Kokai) No. 6-78759 (Patent Literature 1), a serum-free cell culture medium for proliferation of hybridoma, transfectoma, or tumor cell lines, which medium contains human transferrin, human insulin, ethanolamine and sodium selenite in a basal medium, and further contains amino acids, vitamins, cofactors and common mineral salts is disclosed.

In Japanese Laid-open Patent Application (Kokai) No. 2007-77 (Patent Literature 2), a serum free medium for mesenchymal stem cells in which any one or more hormones of cortisol and/or prolactin are added as an active substance in a culture medium for adherent animal cells, and a culture method are disclosed.

In addition, in Japanese Laid-open Patent Application (Kokai) No. 2007-37426 (Patent Literature 3), a serum-free medium for animal mesenchymal stem cell culture in which an antimicrobial peptide having a specific amino acid sequence is added in a basal medium is disclosed.
As described above, a variety of serum-free media for animal cell culture has been disclosed.

However, a highly versatile serum-free medium which can be adapted for somatic stem cells present in adherent cells such as epithelial cells and endothelial cells has not been reported. Therefore, the culture method varies depending on the types of cells, and thus a simple comparison of results of examinations and research has been difficult.

Although plastic vessels are now mainly used for culturing the adherent cells, the adhesion of the cells needs to be improved by a surface treatment such as plasma treatment or the like, or by coating with extracellular matrices of animal origin such as laminin and collagen, and these coatings may affect functions intrinsic to the cells.
Therefore, in order to obtain clear results in various examinations and research using adherent stem cells collected from animal tissues, the development of a culture medium in which the stem cells can maintain their differentiation function and can be intentionally differentiated to construct the tissues under a culture condition in which no unknown factors such as serum are contained and no extracellular matrices are required, as well as the development of an isolation method have been desired.
[Patent Literature 1] Japanese Laid-open Patent Application (Kokai) No. 6-78759
[Patent Literature 2] Japanese Laid-open Patent Application (Kokai) No. 2007-77
[Patent Literature 3] Japanese Laid-open Patent Application (Kokai) No. 2007-37426

### DISCLOSURE OF THE INVENTION

Herein is also described a novel method for isolating cells and culturing the cells, and to provide a cell culture medium by which clear results of research and examinations can be obtained, in which proliferation of the cells can be equal to or better than that in serum and the differentiation of the stem cells can be completely controlled, and which does not require any unknown factors of animal origin nor extracellular matrices.

In order to overcome the above-mentioned problems, the present inventors intensively investigated to discover that, by incubating cells obtained from a tissue in a culture vessel with no coating with an extracellular matrix or no surface treatment such as a plasma treatment, cells such as stem cells can be specifically adhered to the culture vessel and efficiently isolated.
That is, it has been conventionally thought that adherent cells cannot adhere to a culture vessel and culture carrier with no surface treatment of the culture in the absence of the extracellular matrix, and such a culture condition is a culture environment in which the cells are unable to grow. Yet, the present inventors found that cells such as the stem cells can actively produce the extracellular matrix by themselves to proliferate. They further extended the findings to discover a method for isolating only cells of interest, including the stem cells, in the absence of the extracellular matrix and under the condition of no surface treatments.
In addition, they also discovered that, in culturing the isolated cells, by using a medium which common culture additive components namely selenium, insulin, transferrin and ethanolamine (SITE); and lipid soluble essential nutrients such as retinol, linoleic acid, linolenic acid, and α-tocopherol; as well as albumin as a carrier for lipid soluble nutrients are added to a serum-free basal medium in which unknown factors of animal origin such as serum and puietary extracts are absent; and by using a medium in which various growth factors such as epidermal growth factor (EGF), keratinocyte growth factor (FGF-7) and fibroblast growth factor (FGF-2) are further added, the cells such as the stem cells can be reproducibly and efficiently proliferated.

Herein is also described a method for isolating a cell, said method comprising the steps of: dissociating a tissue of animal origin into cells; seeding and incubating said cells in a culture vessel or a culture carrier having a culture surface with no surface treatment; and selecting a cell adhered to said culture surface of said culture vessel or said culture carrier.
Also described is the above-described method, wherein said cell is a stem cell
Herein is also described the above-described method, wherein said stem cell is an epithelial stem cell.
Herein is also described the above-described method, wherein said stem cell is a corneal epithelial stem cell, a conjunctival epithelial stem cell, an oral mucosal epithelial stem cell, or an skin epithelial stem cell.
The present invention also provides a method for culturing a cell, said method comprising the steps of: isolating a cell from a tissue by any one of the above-described methods; and culturing said isolated cell on a medium.
The present invention also provides the above-described method, wherein said medium is a serum-free medium.
The present invention also provides the above-described method, wherein said serum-free medium contains serum albumin.
The present invention also provides the above-described method, wherein said serum-free medium contains insulin, transferrin, sodium selenite, ethanolamine, serum albumin, linoleic acid, linolenic acid, retinol, and α-tocopherol.
The present invention also provides the above-described method, wherein said serum albumin is human serum albumin.
The present invention also provides the above-described method, wherein said human serum albumin is recombinant human serum albumin.
The present invention also provides the above-described method, wherein said serum-free medium contains a differentiation stimulating factor.
The present invention also provides the above-described method, wherein said differentiation stimulating factor is selected from any of EGF, FGF-2 and FGF-7.
Also described is a method for producing a stem cell colony, said method comprising the steps of: isolating a stem cell from a tissue by any one of the above-described methods; and culturing said isolated stem cell in a medium.
Further described is a method for producing a cell sheet, said method comprising the steps of: isolating a cell from a tissue by any one of the above-described methods; and culturing said isolated cell in a medium.
Herein is also described the above-described method, wherein said cell sheet is a stratified epithelial cell sheet.
Herein is also described a method for producing a corneal epithelial cell sheet, said method comprising the steps of: dissociating a corneal limbal epithelial tissue into cells; seeding and incubating said cells in a culture vessel or a culture carrier having a culture surface with no surface treatment; selecting a cell adhered to said culture surface of said culture vessel or said culture carrier; and culturing a corneal epithelial stem cell in a serum-free medium containing EGF or FGF-7.
Herein is also described a method for producing a goblet cell, said method comprising the steps of: dissociating a corneal limbal epithelial tissue into cells; seeding and incubating said cells in a culture vessel or a culture carrier having a culture surface with no surface treatment; selecting a cell adhered to said culture surface of said culture vessel or said culture carrier; and culturing a corneal epithelial stem cell in a serum-free medium containing FGF-2.
Herein is also described a method for producing an skin epithelial cell sheet, said method comprising the steps of: dissociating an epidermal tissue into cells; seeding and incubating said cells in a culture vessel or a culture carrier having a culture surface with no surface treatment; selecting a cell adhered to said culture surface of said culture vessel or said culture carrier; and culturing an skin epithelial cell in a serum-free medium containing EGF.
The present invention provides a medium for stem cell culture containing insulin, transferrin, sodium selenite, ethanolamine, serum albumin, linoleic acid, linolenic acid, retinol and α-tocopherol, which medium does not contain blood serum.
The present invention also provides the above-described medium, wherein serum albumin is human serum albumin.
The present invention also provides the above-described medium, wherein said human serum albumin is recombinant human serum albumin.

### BRIEF DESCRIPTION OF THE DRAWINGS

[Figure 1] Figure 1 shows a micrograph of the isolated corneal epithelial stem cells.
[Figure 2] Figure 2 shows a micrograph of the proliferated corneal epithelial stem cell colony.
[Figure 3] Figure 3 shows figures (photographs) showing the results of nuclear staining of the corneal epithelial stem cell colony with BrdU method and Hoechst33342.
[Figure 4] Figure 4 shows figures (photographs) showing the results of immunostaining of the corneal epithelial stem cell colony with a cytokeratin 3 (CK3) antibody and a cytokeratin 12 (CK12) antibody.
[Figure 5] Figure 5 shows figures (photographs) showing (a) an optical image, (b) an HE stained image (cross-section) and (c) a PAS stained image of the goblet cells obtained by adding FGF-2 to the corneal epithelial stem cells.
[Figure 6] Figure 6 shows (A) a cross-sectional view image (photograph) of the corneal epithelial sheet, (B) a figure showing the delaminated corneal cell sheets and (C) a cross-sectional view (photograph) of the corneal epithelial sheet formed on the amnion, all obtained from the corneal epithelial stem cells.
[Figure 7] Figure 7 shows micrographs of the isolated conjunctival epithelial stem cells.
[Figure 8] Figure 8 shows figures (photographs) showing the results of immunostaining of the conjunctival epithelial stem cell colony with a cytokeratin 4 (CK4) antibody, a cytokeratin 13 (CK13) antibody, BrdU antibody and Nestin antibody.
[Figure 9] Figure 9 shows a figure (photograph) showing (A) an optical image, (B) RT-PCR of MUC5AC and (C) cross-sectional image of the conjunctival epithelial stem cell colony.
[Figure 10] Figure 10 shows a cross-sectional image (photograph) of the conjunctival cell sheet
[Figure 11] Figure 11 shows figures (photographs) showing (A) an optical image of the cell colony obtained by adding EGF to the oral mucosal epithelial stem cells, (B) an optical image of the cell colony obtained by adding FGF-2 to the oral mucosal epithelial stem cells and (C) a cross-sectional image (photograph) of the oral mucosal epithelial cell sheet.
[Figure 12] Figure 12 shows figures (photographs) showing an optical image of (A) the highly-adhesive skin epithelial cell colony, (B) a cross sectional view of the skin epithelial sheet, (C) the result of immunostaining with a cytokeratin1 (CK1) antibody and (D) the result of immunostaining with a cytokeratin10 (CK10) antibody.

### DESCRIPTION OF THE PREFERRED EMBODIMENTS

Originally, it has been thought that adherent cells such as epithelial cells are difficult to be proliferated without attaching to the culture surface and thus an improvement of adhesion by a plasma treatment or by coating with an extracellular matrix, such as collagen, onto the culture surface was required. Yet, particular cells such as somatic stem cells have an ability to produce and secrete the extracellular matrix by themselves and thus the coating of the culture surface with the extracellular matrix, such as collagen, onto the culture surface and the plasma treatment of the culture surface are not required.
Therefore, in the method for isolating cells only cells of interest can be isolated by carrying out adhesion culture of the cells on the culture surface with no surface treatment on which cells other than the cells of interest, such as adherent stem cells, cannot grow. The term "with no surface treatment" means no coating with extracellular matrices such as laminin or collagen and no plasma treatment.

As a desirable culture surface, those with no dissociation of an ionic group on the particle surface, such as --COOH, on the surface of glass, plastics, or the like are desirable. Also, an angle, θ deg, at which a water drop meets the solid surface when the water drop on the surface is observed from the side, is called a contact angle. When such a contact angle is within a range between 0 and 1, adhesion of an adherent animal stem cells are also inhibited. Thus, it is preferred that the contact angle at the culture surface be not less than 1 degree.
Concrete examples of the culture vessel with no surface treatment include FALCON 351143, Kord-Valmark 2901, both of which are made from polystyrene, as well as Celldesk (MS-92132) non-surface treated product of Sumitomo Bakelite Co., Ltd., but are not limited thereto.
Other dishes for suspension culture, which are made from plastics, for example, MS-1160R or MS-1135R of Sumitomo Bakelite Co., Ltd. or non-treated surface Dish 430589 of Corning Incorporated can also be used.
A concrete example of the carrier for culture with no surface treatment includes plastic carriers. Examples of the types of the plastics include polycarbonate, polyalylate, polyethylene terephthalate, polysulfone, polyamine-polystyrene graft copolymers, polystyrene, polyethylene terephthalate, polyethylene terephthalate-polybutylene terephthalate, polyethylene terephthalate, polybutylene terephthalate, segmented polyurethane, polytetrafluoroethylene, poly N-isoacrylamide, polyvinyl alcohol and polyester.
Examples of the form of the carrier include dish-shaped ones, plate-shaped ones, sac-shaped ones, bottle-shaped ones, beads and nonwoven fabrics. As long as a surface where the cells can attach is exposed, it is not restricted.
In addition, types of vessels and carriers which can be used for the culturing can also be obtained in reference to the following: http://idb.exst.jaxa.jp/jdata/02042/199603/19962042000/199603J02042000.html#2.1

Stem cells to which the method of isolation can be applied are preferably those obtained from tissues, organs and internal organs originated from human or mammals other than human. Particularly preferred are undifferentiated type cells such as ES cells and somatic stem cells, more preferred are adherent somatic stem cells, and still more preferred are epithelial stem cells.
Examples of the stem cells include ectoderm stem cells such as neural stem cells, corneal endothelial stem cells, corneal epithelial stem cells, conjunctival epithelial stem cells, skin epithelial stem cells, oral mucosal epithelial stem cells, esophageal epithelial stem cells, thyroid epithelial stem cells, parathyroid epithelial stem cells, thymic epithelial stem cells, enamel epithelial stem cells and follicle; mesoderm stem cells such as renal tubular epithelial stem cells; entoderm stem cells such as gastrointestinal tract epithelial stem cells, biliary tract epithelial stem cells and pancreatic duct epithelial stem cells.
Cells to which the method of isolation can be applied are preferably those obtained from the tissues, organs and internal organs derived from human or mammals other than human, more preferably adherent somatic cells, and still more preferably epithelial cells.

The method for isolating adherent animal stem cells from mammalian tissues and method for culturing the stem cells, as well as method for controlling their differentiation and reconstituting tissues with a growth factor will be actually described in detail below.

First, tissues originated from animals taken out of the body are cut into pieces with scissors or the like, and then dissociated into cells. For the dissociation into the cells, it is preferred to treat with collagenase and trypsin/EDTA solution.
Next, the obtained cells are placed and incubated in the above-mentioned culture vessel with no surface treatment to allow only the stem cells to be selectively attached to the culture vessel. Although it depends on the type of the cells, the incubation is carried out, for example, preferably, at 37°C for 5 to 24 hours. A liquid medium used when the stem cells are attached to the culture vessel may be a buffer solution such as a phosphate buffer or serum-free medium containing various kinds of components described later. Preferably, serum-free medium containing various kinds of components supplemented with a differentiation stimulating substance described later is used.
After the incubation, by removing cells which are not adhered, the stem cells adhered to the vessel can be selected.

As described above, in order to culture and grow the isolated stem cells, an arbitrary medium can be used. Yet, to avoid the influence of the unknown factors, it is preferred to use a serum-free medium, in particular the medium for cell culture according to the present invention.

The medium for cell culture according to the present invention is serum-free and preferably used for culturing adherent animal stem cells. The medium for cell culture according to the present invention is constituted by adding the followings to a serum-free basal medium for animal cell culture. As for the basal medium for animal cell culture, widely used basal media for animal cell culture can be used and DMEM (Dulbecco's Modified Eagle Medium), Ham's F12, DMEM/F-12(1:1), or RPMI1640 or the like can be used.

The medium for cell culture according to the present invention can contain insulin, transferrin, sodium selenite and ethanolamine.
The concentration of each additive is not restricted as long as it is effective for the proliferation of the cells and exhibits no toxicity. And preferably, the concentration of insulin is not less than 0.1 mg/L; that of transferrin is 0.1 mg/L to 10 mg/L; that of sodium selenite is 1 µg/L to 20 µg/L; and that of ethanolamine is 0.5 mg/L to 20 mg/L.
When it comes to roles of each constituting component, insulin is a polypeptide which promotes uptake of glucose and amino acids into the cells; transferrin is a protein for transporting iron which is an essential trace element; selenium is an essential trace element, and ethanolamine is a basic component of a phospholipid which constitutes the cell membrane.

In addition, the medium for cell culture according to the present invention can contain serum albumin, retinol, and α-tocopherol. The concentration of these additives is not restricted as long as it is in a range effective on the proliferation of the cells and exhibits no toxicity. And preferably, the concentration of serum albumin is not less than 0.1 g/L; that of retinol is 0.1 mg/L to 12 mg/ml; and that of α-tocopherol is 0.1 mg/L to 10 mg/ml.
As for roles of these, serum albumin is a protein complex with a fatty acid which solubilizes a fatty acid and promotes uptake of the fatty acid into cells; retinol and α-tocopherol are essential nutrients. It is rather preferred to solubilize retinol and α-tocopherol in the medium. Thus, these can be solubilized in the medium. For instance, there is a method for solubilization by clathrating in or binding to a complex such as cyclodextran and cycloamylose. Also, retinol may be substituted also with vitamin A in a broad sense, that is, retinal, retinoic acid and 3-dehydro form of these and its derivatives.
Further, the medium for cell culture according to the present invention preferably contain linoleic acid and linolenic acid. The concentration of these additives is not restricted as long as it is in a range effective on the proliferation of the cells and exhibits no toxicity. Preferably, the concentration of linoleic acid is not less than 0.5 µg/L; and that of linolenic acid is not less than 0.5 µg/L.
As for roles of these, serum albumin is a protein complex with a fatty acid which solubilizes a fatty acid and promotes uptake of the fatty acid into the cells, and linoleic acid and linolenic acid are essential fatty acids. It is rather preferred to solubilize linoleic acid and linolenic acid in the medium. Thus, these can be solubilized in the medium.

The serum albumin used in the present invention is preferably human serum albumin, and more preferably recombinant human serum albumin. The recombinant human serum albumin (rHSA) is not particularly restricted as long as it is prepared with a genetic recombination technique. For instance, it is preferred that rHSA be sufficiently purified to the extent to be used as a drug (injection solution).
As long as rHSA is an HSA produced by a HSA-producing host and prepared through genetic engineering, it is not restricted. It is preferred to use those which do not substantially contain contaminating components (for example, proteins, polysaccharides and the like) originated from the HSA-producing host, and more preferably those collected and purified, after culturing the rHSA-producing host by a known method, from the culture filtrate, microbial cells, or cells with a corresponding known separation method and purification method are used. Examples of the rHSA producing host include yeast such as the genus *Saccharomyces* and the genus *Pichia*, animal cells and transgenic animals. With regard to rHSA, Japanese Laid-open Patent Application 2005-204539 describes in detail.

In addition, for the purpose of eliminating cytotoxicity by free radicals generated from oxygen and cells themselves because of no serum, one or more types of superoxide dismutase (SOD), catalase and gluthatione peroxidase may be added. The concentration of these additives is not restricted as long as it is in a range effective on the proliferation of the cells and exhibits no toxicity. Preferably, the concentration of SOD is 0.25 mg/L to 25 mg/ml; that of catalase is 0.1 mg/L to 10 mg/L; and that of gluthatione peroxidase is 0.1 mg/L to 10 mg/L. These can be substituted with other enzymes having other antioxidant effects or chemical substances having the antioxidant effects such as ascorbic acid, and their derivatives.

Additionally, for the purpose of promoting cell proliferation, triiodothyronine, putrescine and progesterone may be added. The concentration of these additives is not restricted as long as it is in a range effective on the proliferation of the cells and exhibits no toxicity. Preferably, the concentration of triiodothyronine is 0.2 µg/L to 200 µg/L; that of putrescine is 10 µM to 1 mM; and that of progesterone is 2 nM to 200 nM.

By adding various differentiation stimulating factors to the thus prepared medium for cell culture, the differentiation of the stem cells can be controlled. Isolation, proliferation and differentiation of various stem cells and the production of cell sheets will be described below.

In the case of the corneal epithelial stem cells, by isolating corneal limbal epithelial tissues and subjecting them to the method of isolation, the corneal epithelial stem cells can be obtained.
Whether the isolated cells are the corneal epithelial stem cells can be confirmed by their ability to provide cells in which expression of cytokeratin 3 (CK3) and cytokeratin 12 (CK12), which are differentiation markers of the corneal epithelial cells, are observed, by cell division (abilities to differentiate into other cells and to proliferate) and their ability to form stratified corneal epithelial tissue which is positive for these corneal epithelial cell markers (ability to form tissue). The confirmation of the expression of the differentiation markers can be carried out by analysis of mRNA such as RT-PCR or Northern blot and analysis of protein such as antibody staining. The ability to proliferate can be confirmed by, in addition to BrdU method and immunostaining with Ki67, visibly recognizing chromosomes under a microscope when the cultured cells are dividing. The ability to form tissue can be confirmed by examining formation of the stratified epithelial tissue and expression of the differentiation markers when the cells are cultured.
The obtained corneal epithelial stem cells can be differentiated into desired cells by adding differentiation stimuli within the range of the differentiation ability of the stem cells. For instance, by adding EGF and FGF-7 to the medium for stem cell culture and culturing, the corneal epithelial stem cells can be differentiated into the corneal epithelial cells. And by adding FGF-2 to the corneal epithelial stem cells, they can be differentiated into the goblet cells.
And, the corneal epithelial stem cells can be obtained as a cell colony. The corneal epithelial stem cells can also be obtained as a corneal epithelial cell sheet by proliferating them in the form of a sheet and removing the thus formed sheet with an EDTA treatment or the like. The corneal epithelial cell sheet can be produced on an amnion. The obtained corneal epithelial cell sheet can be stratified.
The obtained corneal epithelial cell sheet can be applied to a therapy in regenerative medicine for intractable ocular surface disorder such as an intractable corneal epithelial disorder.

In the case of conjunctival epithelial stem cells, by collecting conjunctiva from a piece of the scleral cornea and subjecting them to the method for isolation,-the conjunctival epithelial stem cells can be obtained.
Whether the isolated cells are the conjunctival epithelial stem cells can be confirmed by their ability to provide cells in which expression of cytokeratin 4 (CK4) and cytokeratin 13 (CK13), which are differentiation markers of the corneal epithelial cells, are observed, by cell division (abilities to differentiate into other cells and to proliferate) and their ability to form stratified conjunctival epithelial tissue which is positive for these conjunctival epithelial cell markers (ability to form tissue).
The obtained conjunctival epithelial stem cells can be differentiated into desired cells by adding differentiation stimuli in the range of the differentiation ability of the stem cells. For instance, by adding EGF and FGF-7 to the medium for stem cell culture and culturing, the conjunctival epithelial stem cells can be differentiated into the conjunctival epithelial cells. And, by adding FGF-2 to the corneal epithelial stem cells, they can be differentiated into the goblet cells.
And, the conjunctival epithelial stem cells can be obtained as a cell colony. The conjunctival epithelial stem cells can also be obtained as a conjunctival epithelial cell sheet by proliferating them in the form of a sheet and removing the thus formed sheet with an EDTA treatment or the like. The conjunctival epithelial cell sheet can be produced on an amnion. The obtained conjunctival epithelial cell sheet can be stratified.
The obtained conjunctival epithelial cell sheet can be applied to reconstruction of epithelia after enucleation of tumors, and formation of filtration blebs in a glaucoma surgery.

In the case of oral mucosal epithelial stem cells, by collecting the oral mucosal epithelial cells and subjecting them to the method for isolation, the oral mucosal epithelial stem cells can be obtained.
Whether the isolated cells are the oral mucosal epithelial stem cells can be confirmed by their ability to provide cells in which expression of CK3, CK4, and CK13, which are differentiation markers of the oral mucosal epithelial cells, are observed, by cell division (abilities to differentiate into other cells and to proliferate) and their ability to form stratified mucosal epithelial tissue which is positive tor these mucosal epithelial cell markers (ability to form tissue).
The obtained oral mucosal epithelial stem cells can be differentiated into desired cells by adding differentiation stimuli in the range of the differentiation ability of the stem cells. For instance, by adding EGF and FGF-7 to the medium for stem cell culture and culturing, the oral mucosal epithelial stem cells can be differentiated into the oral mucosal epithelial cells. And, by adding FGF-2 to the corneal epithelial stem cells, they can be differentiated in to the goblet cells.
And, the oral mucosal epithelial stem cells can be obtained as a cell colony. The oral mucosal epithelial stem cells can also be obtained as an oral mucosal epithelial cell sheet by proliferating them in the form of a sheet and removing the thus formed sheet with an EDTA treatment or the like. The oral mucosal epithelial cell sheet can be produced on an amnion. The obtained oral mucosal epithelial cell sheet can be stratified.
The obtained oral mucosal epithelial cell sheet can be applied to reconstruction of oral mucosal epithelia and a therapy for bilateral ocular surface disorder or the like.

In the case of skin epithelial stem cells, by collecting the skin epithelial cells and subjecting them to the method for isolation, the skin epithelial cells can be obtained. And, the skin epithelial stem cells can also be obtained as a cell colony. A skin epithelial cell sheet can be obtained by proliferating the obtained skin epithelial stem cells in the form of a sheet and removing the thus formed sheet with an EDTA treatment or the like. The skin epithelial cell sheet can also be produced on an amnion. The obtained skin epithelial cell sheet can be stratified.
The obtained skin epithelial cell sheet can be applied to reconstruction of skin and a therapy for severe bum or the like.
Whether the isolated cells is the skin epithelial cells can be confirmed by their ability to provide cells in which expression of cytokeratin 1 (CK1) and cytokeratin 10 (CK10), a keratin pattern specific to skin epithelial cells which is not expressed in the skin epithelial stem cells themselves, are observed, by cell division (abilities to differentiate into other cells and to proliferate) and their ability to form stratified skin epithelial tissue (ability to form tissue). Further, the ability of skin epithelial cells to differentiate can be checked by change of the cells into other kinds of cells which are not known in current knowledge.
As described above, the corneal epithelial stem cells, conjunctival epithelial stem cells, oral mucosal epithelial stem cells and skin epithelial stem cells are illustrated. And other stem cells or cells can be isolated in the same manner.

### EXAMPLES

The present invention will be described in more detail below in reference to the examples; however, the scope of protection according to the present invention is not limited thereto.

SITE media supplement (SIGMA) was added to serum-free basal medium DMEM/F-12 (1:1) so as to attain an insulin concentration of 10 mg/L, a transferrin concentration of 5.5 mg/L, a sodium selenite concentration of 5 µg/L and an ethanolamine concentration of 2 mg/L.
Also, bovine serum albumin (Invitrogen) 2.5 g/L, linoleic acid 5 µg/L (SIGMA), linolenic acid 5 µg/L (SIGMA), retinol 100 µg/L (SIGMA) and α-tocopherol 1 mg/L (SIGMA) were added.
Further, superoxide dismutase (SOD) 2.5 mg/L (SIGMA), catalase (SIGMA), gluthatione peroxidase 1 mg/L (Wako Pure Chemical Industries, Ltd.) were added.
Furthermore, triiodothyronine 20 µg/L, putrescine 100 µM (SIGMA), progesterone 20 nM (MPB Biomedicals, Inc.) were added to obtain a medium for stem cell culture (A), which was used in the examples below.
In addition, SITE media supplement (SIGMA) was added to DMEM/F-12 basal medium (SIGMA) so as to attain an insulin concentration of 10 mg/L, a transferrin concentration of 5.5 mg/L, a sodium selenite concentration of 5 µg/L and an ethanolamine concentration of 2 mg/L. Recombinant human serum albumin (Mitsubishi Tanabe Pharma Corporation) 2.5 g/L, retinol 100 µg/L (SIGMA), α-tocopherol 1 mg/L (SIGMA), superoxide dismutase (SOD) 2.5 mg/L(SIGMA), catalase (SIGMA) and gluthatione peroxidase 1 mg/L (Wako Pure Chemical Industries, Ltd.) were added. Triiodothyronine 20 µg/L, putrescine 100 µM (SIGMA) and progesterone 20 nM (MPB Biomedicals, Inc.) were added to obtain a medium for stem cell culture (B), which was used in the examples below. Since recombinant human albumin preparations manufactured by Mitsubishi Tanabe Pharma Corporation contained linoleic acid and linolenic acid, these acids were not newly added when the medium was prepared.

### [Example 1]

### (Isolation of corneal epithelial stem cells)

The production of a suspension culture vessel for stem cells was carried out in accordance with the method described in Japanese Patent Application No. 2005-369270 (Japanese Laid-open Patent Application (Kokai) No. 2007-166977) by dissolving polyhydroxyethyl methacrylate described in the centrifuge tube for living cell separation in ethanol, and coating the resultant to a commercially available plastic culture vessel for suspension culture, followed by air-dry. This suspension culture vessel for stem cells may be used for the purpose of preventing adhesion of the stem cells that is not intended by the researchers.

After conjunctival tissues were removed from imported human cornea for research obtained from the Eye Bank Association of America, corneal limbal epithelial tissues where corneal epithelial stem cells were considered to exist were excised with scissors. The excised corneal limbal epithelial tissues were placed in the suspension culture vessel for stem cells filled with 10 ml of a serum-free medium for culturing adherent animal cells supplemented with collagenase TYPE IA (SIGMA) 0.02%, and cultured overnight under a condition at 37°C and 5%CO₂ to dissolve the tissue, thereby obtaining cell aggregates.

The obtained cell aggregates were collected in a centrifuge tube STEMFULL (Sumitomo Bakelite Co., Ltd.) and centrifuged at 1200 rpm/min. The serum-free medium for culturing adherent animal stem cells containing collagenase was removed. And then the cells were washed with PBS(-) and centrifuged at 1200 rpm/min to remove PBS(-). Subsequently, the resultant was dispersed to single cells using trypsin/EDTA and then the washing with PBS(-) were repeated three times to remove trypsin/EDTA. The cells were suspended in 1mL of the medium for stem cell culture (A) to which EGF was added to a final concentration of 20 ng/ml. The cell density of the cell suspension was measured using Coulter Counter Z1 (Beckman Coulter, Inc.).

After the measurement of the number of cells, the cell suspension was diluted to a cell density of 1000 cells/mL using the medium for stem cell culture (A) in the suspension culture vessel for stem cells. This cell suspension containing the stem cells was seeded at 1000 cells/well on a 24-well non-treated plate (FALCON) and cultured overnight under a condition at 37°C and 5% CO₂.

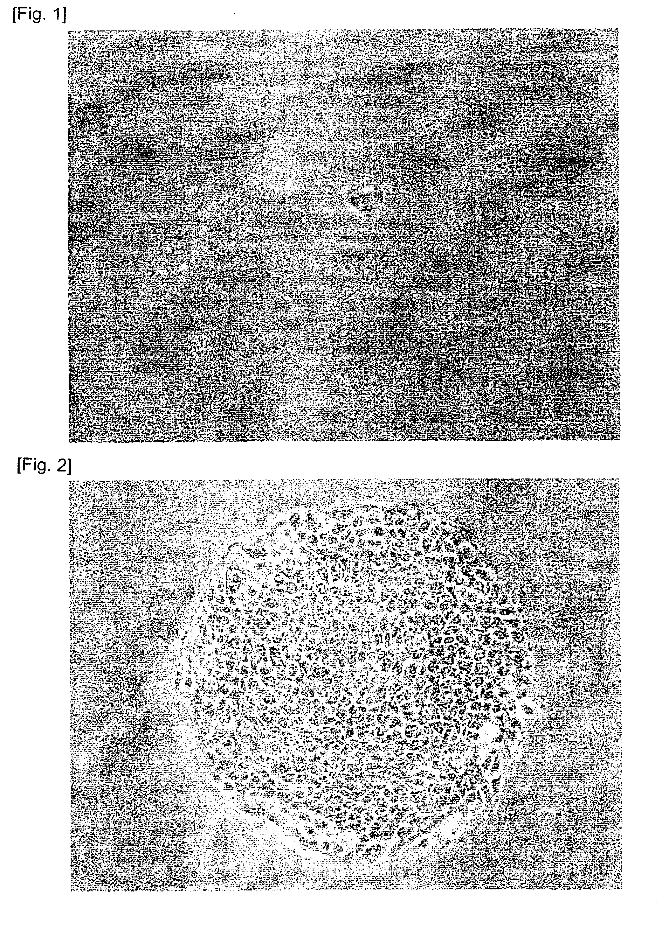

Thereafter, the culture supernatant was removed and the cells were washed twice with PBS(-) and then washed once with the medium for stem cell culture (A) to remove cells which were not adhered to the non-coated plastic plate, thereby isolating only the corneal epithelial stem cells shown in Figure 1.

### [Example2]

### (Test for differentiation into the corneal epithelial cells by a growth factor)

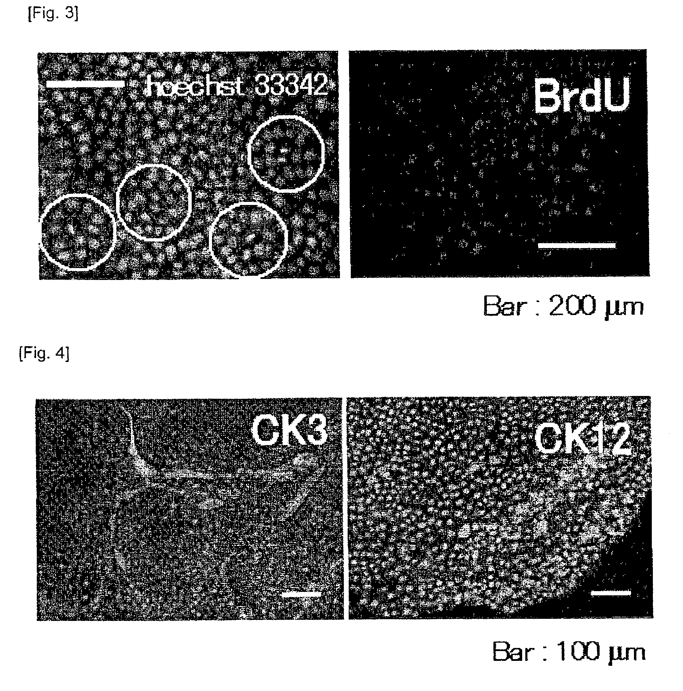

Epidermal growth factor (EGF: obtained from Wako Pure Chemical Industries, Ltd.) was added to the medium for stem cell culture (A) so as to attain a concentration of 20 ng/mL. The culturing of the isolated corneal epithelial stem cells was carried out under a condition at 37°C and 5% CO₂. The isolated corneal epithelial stem cells began vigorously to divide to form a round shaped cell population (colony) originated from a single cell in about one week as shown in Figure 2. BrdU (Roche) uptake test (BrdU method) and nuclear staining with Hoechst33342 (Invitrogen), both by which the ability to proliferate can be checked, were carried out to this colony. The results of those are shown in Figure 3. It can be seen that corneal epithelia cells are vigorously proliferating because the colony is positive for a FITC-Conjugated anti-BrdU antibody (Roche), which is an antibody for detecting BrdU taken up by cells in which DNA was replicated and the chromosome appearing during cell division can be confirmed. The morphology of the colony shows a cobblestone appearance typical for epithelial cells. Protein expression of CK3 and CK12, which is a keratin expression pattern specific to corneal epithelia, was examined by an immunostaining method. An immunological antibody reaction was carried out using a primary antibody AE-5 (Progen) for detecting CK3 and a primary antibody N-16 (Santa Cruz Biotech) for detecting CK12. Thereafter, a secondary antibody Alexa Fluor 488 goat anti-mouse IgG (H+L) (Alexa 488: Invitrogen) was allowed to react. As a result, the expression of CK3 and CK12, which is a cytokeratin pattern specific to the corneal epithelial cells, was confirmed as shown in Figure 4. From these results, it was confirmed that the corneal epithelial stem cells collected from the corneal limbal epithelial tissue had the ability to differentiate into the corneal epithelial cells.

### [Example 3]

### (Test for differentiation into the goblet cells by a growth factor)

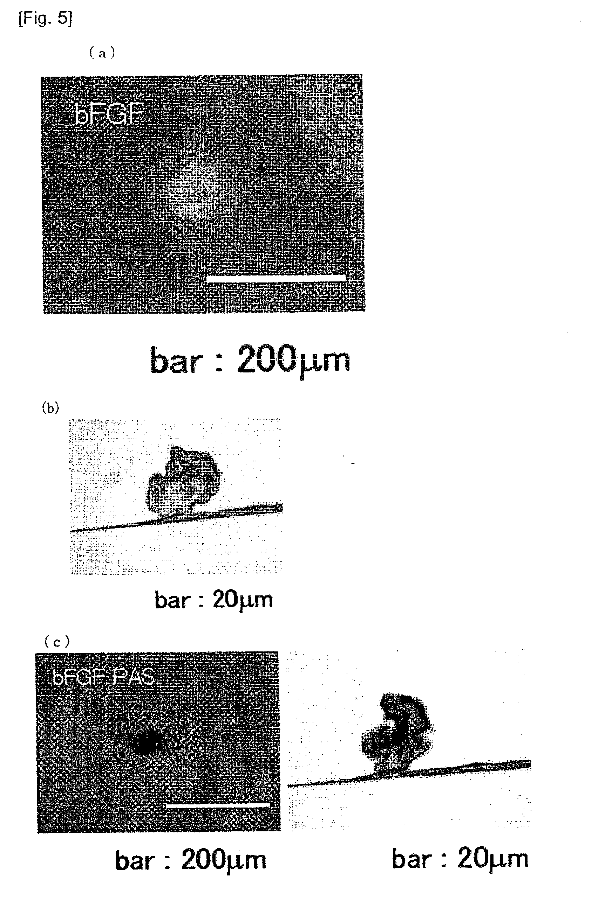

A fibroblast growth factor (FGF-2: obtained from Wako Pure Chemical Industries, Ltd.) was added to the medium for stem cell culture (A) so as to attain a concentration of 40 ng/mL. The culturing of the isolated corneal epithelial stem cells was carried out under a condition at 37°C and 5% CO₂.
The isolated corneal epithelial stem cells began vigorously to proliferate and, in about 3 to 5 days, a middle portion elevated to form a protrusion. And a colony originated from a single cell with a specific morphology appeared (Figure 5a). As seen in a cross-sectional view of this protrusion in the middle, it contained a small amount of cell components. Thus, the colony was proven not to be a cell clump and to be rather a cluster of extracellular matrices (Figure 5b). And then PAS staining which shows positive in the presence of the goblet cells was carried out, and the cells were found to be positive (Figure 5c). Thus, the corneal epithelial stem cells were able to be controlled to differentiate into the goblet cells.

### [Example4]

### (Test for tissue formation by the stem cells)

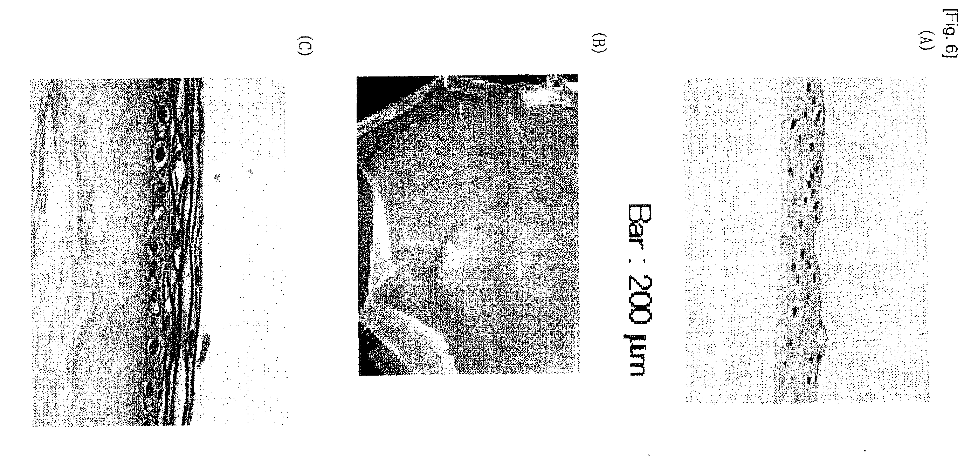

Epidermal growth factor (EGF: obtained from Wako Pure Chemical Industries, Ltd.) was added to the medium for stem cell culture (A) so as to attain a concentration of 20 ng/mL. The culturing of the isolated corneal epithelial stem cells was carried out under a condition at 37°C and 5% CO₂ for 3 weeks. After cultured for 3 weeks, the cells were fixed with 10% formalin and embedded in paraffin. And then the cross section was checked by Hematoxylin-Eosin staining method as shown in Figure 6A. The stem cells formed a corneal epithelial tissue stratified with about five layers. Additionally, a photograph of the corneal epithelial sheet obtained by dissociating the corneal epithelial stem cells cultured for 3 weeks is shown in Figure 6B.

### [Example 5]

### (Production of corneal epithelial sheets)

Subsequently, the production of the corneal epithelial sheets on the amnion using the medium for stem cell culture (B) was carried out. The amnion was obtained from a donor expected to have a caesarean section. The donor was informed of and consented to a use of the amnion for ophthalmic research and treatment. The amnion which was stored frozen after aseptic collection and from which chorionic villus was removed using forceps was used. The amnion was washed with PBS(-) and incubated in a 0.02% EDTA/PBS(-) solution in a 50 ml-centrifuge tube manufactured by Corning Incorporated at 37°C in an incubator for 2 hours. Thereafter, amniotic epithelia were removed with a cell scraper manufactured by Corning Incorporated. Only the amniotic substrate was collected and then was attached to a TransWell 6well Plate manufactured by Corning Incorporated.

From a piece of the adult scleral cornea provided as a research material by the Eye Bank Association of America, the corneal limbal epithelial tissues located around the cornea, in which corneal epithelial stem cells are abundantly present, were pinched with forceps and removed with scissors, thereby obtaining a tissue piece. Onto the amniotic substrate immersed in the medium for stem cell culture (B) added with EGF (Wako Pure Chemical Industries, Ltd.) to a concentration of 20 ng/mL, the corneal limbal tissue with a size of about 1 mm×1 mm was placed. The culture was continued for 4 weeks while the medium was changed once in 2 to 3 days, thereby forming the corneal epithelial cell sheet. The example thereof is shown in Figure 6C.

### [Example 6]

### (Isolation of the conjunctival stem cells and production of the cell sheet)

The conjunctiva was collected from imported human cornea for research obtained from the Eye Bank Association of America. The obtained conjunctiva was excised with scissors. The excised conjunctiva was placed in the suspension culture vessel for stem cells filled with 10 ml of the medium for stem cell culture (A) added with 0.02% collagenase TYPE IA (SIGMA) and cultured overnight under a condition at 37°C and 5% CO₂ to dissolve the tissue, thereby obtaining an aggregate of cells.

The obtained cell aggregate was collected in a centrifuge tube STEMFULL (Sumitomo Bakelite Co., Ltd.) and subjected to centrifugation at 1200 rpm/min. After the medium for stem cell culture (A) containing collagenase was removed, the cells were washed with PBS(-) and again centrifuged at 1200 rpm/min to remove PBS(-). Thereafter, the cells were treated with 0.05% trypsin/0.02% EDTA for 10 minutes to disperse the cells to single cells. And then the washing with PBS(-) was carried out three times to remove trypsin/EDTA and the cells were suspended in 1 ml of the medium for stem cell culture (A) to which EGF (Wako Pure Chemical Industries, Ltd.) was added to a concentration of 20 ng/mL. The cell density of the cell suspension was measured using a Coulter Counter Z1 (Beckman Coulter, Inc.).

After the measurement of the number of the cells, the cells were diluted in the suspension culture vessel for stem cells using the medium for stem cell culture (A) to which EGF (Wako Pure Chemical Industries, Ltd.) was added to a concentration of 20 ng/mL so as to attain a cell density of 1000 cells/mL. This cell suspension containing the stem cells was seeded on a 24 well non-treated plate (FALCON) at 1000 cells/well and cultured overnight under a condition at 37°C and 5% CO₂.

Thereafter, the culture supernatant was removed and the cells were washed twice with PBS(-) and washed once with the medium for stem cell culture (A) to remove cells which were not adhered to the non-coated plastic plate, thereby isolating only the conjunctival stem cells.

The adherent cells were cultured in the medium for stem cell culture (A) to evaluate protein expression, proliferation ability, and ability to form tissue.

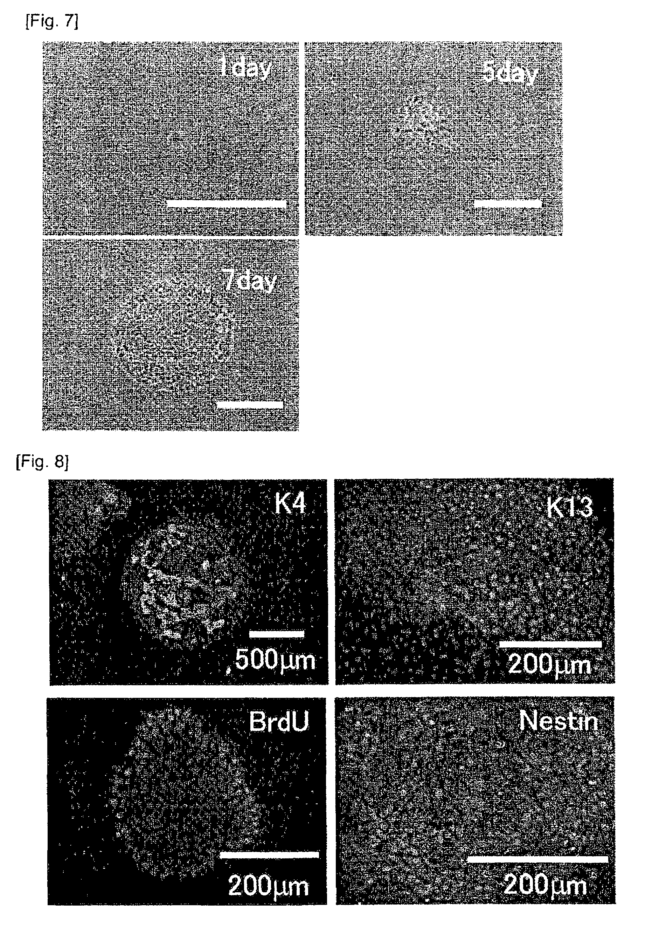

As a result, 0.6% of the seeded cells originated from the conjunctiva were adhered. Among them, 27% of the cells formed a colony and the cells assumed an epithelial-like morphology (Figure 7).
Continuously, expression of the protein was analyzed to identify the cells. To check protein expression of CK4 and ACK13, which is a cytokeratin expression pattern specific to conjunctiva epithelia, immunostaining was carried out using antibodies consisting of mouse anti-human Cytokeratin4 antibody (ICN Pharmaceuticals) and mouse anti-human cytokeratin 13 antibody (American Research Products), which antibodies were allowed to react with a secondary antibody, Alexa, Fluor 488 goat anti-mouse IgG (Invitrogen). In addition, a undifferentiated cell maker protein, Nestin was stained with a mouse anti-Nestin (BD PharMingen) and detected with a secondary antibody Alexa Fluor 488 goat anti-mouse IgG. As a result, it was shown that these cell colonies with the epithelial-like morphology expressed CK4 and CK13, which is the cytokeratin expression pattern specific to conjunctival epithelia and were thus the conjunctival epithelial cells. Also, the cells were positive for the undifferentiated cell maker, nestin.
On the other hand, the cells were negative for the corneal epithelia-specific markers, CK3 and CK12. Also, by BrdU method, it was seen that the cells forming the colony had a proliferation ability (Figure 8).
It was seen that the colony can be subcultured up to P2 and had an ability to self-replication.

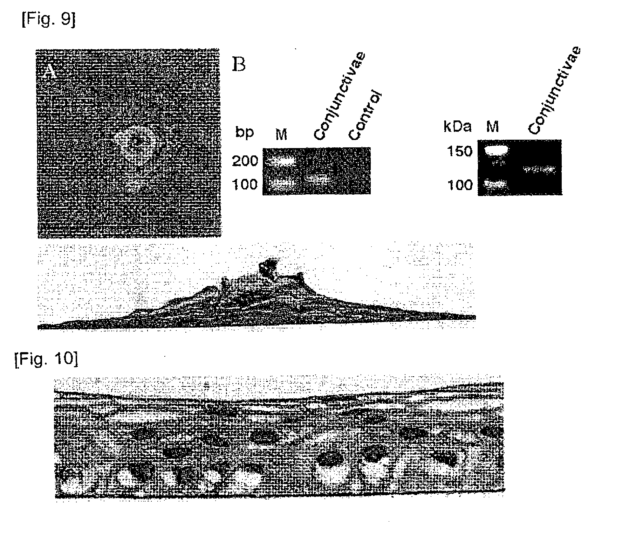

When the cells were further cultured, amorphous secretions were seen in the middle of the colony originated from a single cell. This cell colony was positive for a goblet cell-specific marker MUC5AC. As seen in a cross sectional view of the colony subjected to PAS staining, the cells were PAS positive (mucin production) and differentiated into the goblet cells (Figure 9). Expression of MUC5AC was examined by RT-PCR using primers shown in SEQ ID NOs. 1 and 2.

The colony originated from a single cell was removed in the form of a sheet using EDTA. And then, a conjunctival sheet with 5 to 6 layers was produced on a 6-well transwell under a condition in which neither 3T3 feeder cells nor sera were present.
There was a difference in the ability to form tissue among the cell colonies. The ability was able to be classified into three types from the strongest to the weakest. The photograph shows the one with the highest ability to form tissue (Figure 10).

As described above, the undifferentiated cell including the human conjunctival epithelial stem cell and a transient amplifying cell can be collected to produce a three-dimensional conjunctival epithelial cell sheet originated from a single cell in a serum-free, feeder Cell-free medium.

### [Example7]

### (Isolation of oral mucosal epithelial stem cells and production of cell sheets)

An oral mucosal epithelial tissue no longer necessary after the removal of teeth was obtained with donor's consent. The oral mucosal epithelial tissue was subjected to an enzyme treatment in a tissue-dissolving solution in which DispaseII (Roche) was added to DMEM/F12 (SIGMA) to 1.2 U/ml at 4°C for not less than 4 hours. Thereafter, only an epithelial cell layer was manually removed from the oral mucosal epithelial tissue using forceps and collected in a centrifuge tube STEMFULL (Sumitomo Bakelite Co., Ltd.).

The cells were washed with PBS(-) and centrifuged at 1200 rpm/min to remove PBS(-). Thereafter, the cells were treated with 0.05% trypsin/0.02% EDTA for 10 minutes to disperse the cells to single cells. And then the washing with PBS(-) was repeated three times to remove trypsin/EDTA. The cells were then suspended in 1 ml of the medium for stem cell culture (A) to which a growth factor was not added. The cell density of the cell suspension was measured using a Coulter Counter Z1 (Beckman Coulter, Inc.).

After the measurement of the number of the cell, the cells were diluted in the suspension culture vessel for stem cells using the medium for stem cell culture (A) so as to attain a cell density of 1000 cells/mL. This cell suspension containing the stem cells was seeded on a 24 well non-treated plate (FALCON) at 1000 cells/well and cultured overnight under a condition at 37°C and 5% CO₂.

Thereafter, the culture supernatant was removed and the cells were washed twice with PBS(-) and washed once with the medium for stem cell culture (A) to remove cells which did not adhere to the non-coated plastic plate, thereby isolating only oral mucosal epithelial stem cells.

The adherent cells were cultured in the medium for stem cell culture (A) to evaluate their ability to form colony and ability to produce secretions upon stimulus of FGF-2.

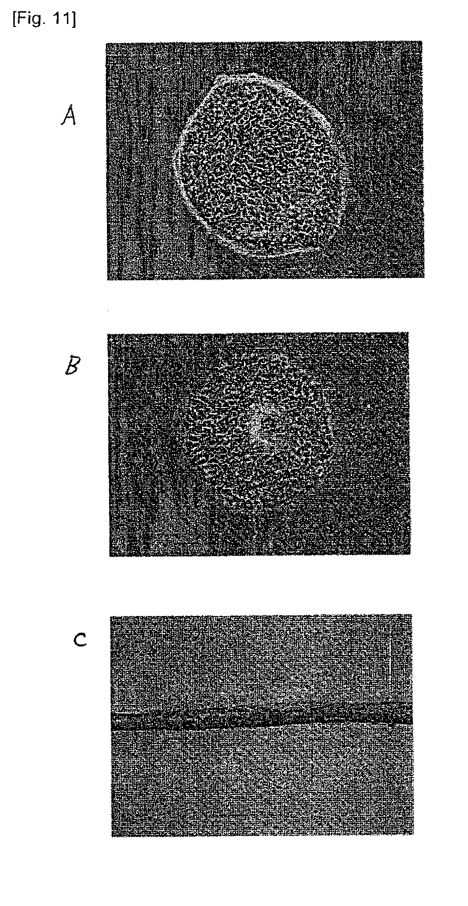

When the culture was continued in the medium for stem cell culture (A) to which EGF (Wako Pure Chemical Industries, Ltd.) was added so as to attain a concentration of 20 ng/mL, an epithelial-like colony shown in Figure 11(A) was obtained. Also, when the culture was continued in the medium for stem cell culture (A) to which FGF-2 (Wako Pure Chemical Industries, Ltd.) was added so as to attain a concentration of 40 ng/mL, a goblet cell-like colony shown in Figure 11(B) which releases secretions was obtained.

Next, the production of oral mucosal epithelial sheets using the medium for stem cell culture (A) was attempted. The amnion was obtained and prepared in the same manner as described in Example 5.

The oral mucosal epithelial cells collected from the oral mucosal epithelial tissue using collagenase and trypsin were suspended in the medium for stem cell culture (A) to which EGF (Wako Pure Chemical Industries, Ltd.) was added to a concentration of 20 ng/mL. The oral mucosal epithelial cells were seeded on the amniotic substrate at a cell density of 100,000 cells/well. The culture was continued for 3 weeks while the medium was changed once in two to three days, thereby producing the oral mucosal epithelial sheet. The example thereof is shown Figure 11(C).

### [Example8]

### (Isolation of highly adherent skin epithelial stem cell colonies)

Skin no longer necessary after a plastic and reconstructive surgery was obtained with donor's consent. A skin tissue was subjected to an enzyme treatment in a tissue-dissolving solution to which DispaseII (Roche) was added to DMEM/F12 (SIGMA) added to 1.2 U/ml at 4°C for not less than 4 hours. Thereafter, only an epidermal cell layer was manually removed from the skin tissue using forceps and collected in a centrifuge tube STEMFULL manufactured by Sumitomo Bakelite Co., Ltd.).

The cells were washed with PBS(-) and centrifuged at 1200 rpm/min to remove PBS(-). Thereafter, the resultant was dispersed to single cells using trypsin/EDTA and then the washing with PBS(-) were repeated three times to remove trypsin/EDTA. The cells were suspended in 1mL of the medium for stem cell culture (A) to which EGF was added to a final concentration of 20 ng/ml. The cell density of the cell suspension solution was measured using Coulter Counter Z1 (manufactured by Beckman Coulter, Inc.). After the measurement of the number of cells, the cell suspension was diluted to a cell density of 100,000 cells/mL using the medium for stem cell culture (A).

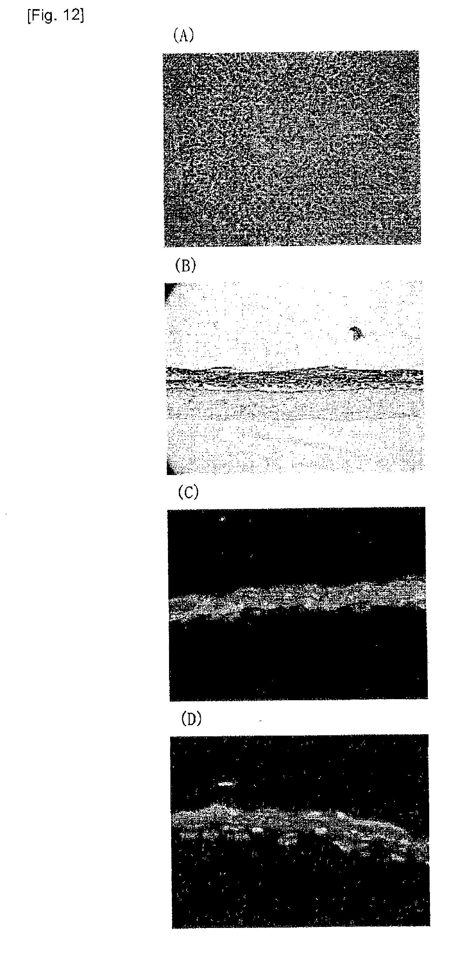

This cell suspension was seeded at 2500 cells/cm² on Valmark, a non-treated 60 mm petri dish and cultured overnight under a condition at 37°C and 5% CO₂. Thereafter, the culture supernatant was removed and the cells were washed twice with PBS(-) and then washed once with the medium for stem cell culture (A) to remove cells which did not adhered to the non-coated plastic dish, thereby isolating only cells with a high adhesion ability, which ability was seen in the epithelial stem cells. The culturing was continued in the medium for stem cell culture (A) to which EGF (Wako Pure Chemical Industries, Ltd.) was added to a concentration of 20 ng/mL, and an epithelial-like colony was obtained as shown in Figure 12A.
The skin epithelial cells were seeded at a cell density of 100,000 cells/well on the amniotic substrate attached to a TransWell 6well Plate manufactured by Corning Incorporated, which plate was prepared in the same manner as described in Example 5. The culturing was continued for 3 weeks while the medium was changed once in two to three days, thereby producing a cell sheet (Figure 12B).
After the cells were fixed with 10% formalin and embedded in paraffin, evaluation of the cross section of the cell sheet with Hematoxylin-Eosin staining method was carried out. The cell sheet was stratified. The cell sheet having a basal layer, whose surface assumed a keratinization-like appearance, was confirmed. For tissue evaluation of the cell sheet, using cytokeratin 1 (CK1) AE1 (PROGEN Biotechnik GmbH) and cytokeratin 10 (CK10) VIK-10 (BioVendor Laboratory Medicine, Inc), which is a keratin expression pattern specific to epidermal epithelia, immunostaining was carried out. For secondary staining, Alexa, Fluor 488 goat anti-mouse IgG (Invitrogen) was allowed to react. As a result, the cell sheet produced from the skin epithelial cells expressed CK1 and CK10, which is the cytokeratin pattern specific to epidermal epithelia, and was proven to be the skin epithelial cell sheet (Figure 12C, D).

### INDUSTRIAL APPLICATIBILITY

By using this method, it is possible toreadily and efficiently isolate cells, particularly stem cells from tissues. Conventionally, a surface treatment such as a plasma treatment of a culture surface or coating with an extracellular matrix, both of which promote adhesion is required in cell culture. Those are not required because the stem cells by themselves are able to produce and secret the extracellular matrix. Thus, influences of the surface treatment and coated the extracellular matrix can be eliminated. In addition, since a serum-free medium used for cell culture does not contain unknown factors of animal origin, it is not only beneficial for eliminating an unknown pathogen but also enables high reproducibility and clear experimental and test results to be attained.
The stem cells which can be prepared have significant industrial applicability. The stem cells obtained can produce a tissue composed of several millions of cells from a single cell. There is a fair possibility that a virus targeting and infecting these large amount of cells exists. That is thought to be one of the best ways for the virus to infect and for its own DNA or RNA to proliferate. Because of that, there is a possibility that some viruses may have a mechanism to distinguish the stem cells and to infect them. Also, for the stem cells themselves, virus infection could be a serious threat. Because of that, it is expected that the stem cells have a defense mechanism against infection. Additionally, there are thought to be many diseases caused by an abnormal function of the stem cells themselves. Yet, these are out of reach from current knowledge of human beings. This is because a means for readily isolating the stem cells in the absence of serum was not thus far available. However, by the present disclosure, the knowledge can be obtained any time. The present disclosure is of extremely great value as a method for preparing reagents and samples for research which reagents and samples are used in development of drugs for infectious diseases and cancers, or the like.
In addition, the epithelial stem cells vigorously secrete mucin upon a stimulus of a specific growth factor or secrete a large amount of specific extracellular matrices. It is expected that the stem cells possess a metabolic pathway for these. To clarify such a metabolic pathway has a significant impact on yield efficiency upon industrial use of recombinant yeast.
Furthermore, the medium according to the present invention is superior in terms of cell's ability to proliferate, compared with cases where serum is used. In the presence of an appropriate growth factor, the cells can be cultured while fulfilling their ability to differentiate into tissues or other cells. Addition of the growth factor alone enables differentiation of the cells to be completely controlled and allows body tissues to be formed. In these formed body tissues, the possibility of unknown infectious diseases can be eliminated, compared with cases where serum is used. Thus, the cells can be used for regenerative medicine, for example, a treatment of intractable corneal epithelial disorder and the like.

## Claims

1. A medium for stem cell culture consisting of
(a) a serum free basal medium for animal cell culture selected from the group consisting of DMEM, Ham's F12, DMEM/F-12 (1:1) or RPMI1640;
(b) insulin, transferrin, sodium selenite, ethanolamine, serum albumin, linoleic acid, linolenic acid, retinol and α-tocopherol
(c) and optionally one or more selected from the group consisting of SOD, catalase, glutathione peroxidase, triiodothyronine, putrescine, progesterone,
(d) and further optionally one or more selected from the group consisting of EGF, FGF-7 and FGF-2;
which medium does not contain blood serum.

2. The medium according to claim 1, wherein said serum albumin is human serum albumin.

3. The medium according to claim 2, wherein said human serum albumin is recombinant human serum albumin.

4. A method for culturing an epithelial stem cell, said method comprising the step of culturing the cell in the medium according to any one of claims 1 to 3.

5. The method according to claim 4, wherein said epithelial stem cell is a corneal epithelial stem cell, a conjunctival epithelial stem cell, an oral mucosal epithelial stem cell, or a skin epithelial stem cell.

## Patentansprüche

1. Medium für eine Stammzellkultur, bestehend aus
(a) einem serumfreien Basalmedium für eine Tierzellkultur, ausgewählt aus der Gruppe bestehend aus DMEM, Ham's F12, DMEM/F-12 (1:1) oder RPMI1640;
(b) Insulin, Transferrin, Natriumselenit, Ethanolamin, Serumalbumin, Linolsäure, Linolensäure, Retinol und α-Tocopherol,
(c) und gegebenenfalls einem oder mehrere Vertreter, ausgewählt aus der Gruppe bestehend aus SOD, Katalase, Glutathion-Peroxidase, Triiodothyronin, Putrescin, Progesteron,
(d) und ferner gegebenenfalls einem oder mehrere Vertreter, ausgewählt aus der Gruppe bestehend aus EGF, FGF-7 und FGF-2;
wobei das Medium kein Blutserum enthält.

2. Medium gemäß Anspruch 1, wobei das genannte Serumalbumin humanes Serumalbumin ist.

3. Medium gemäß Anspruch 2, wobei das genannte humane Serumalbumin rekombinantes, humanes Serumalbumin ist.

4. Verfahren zur Kultivierung einer epithelialen Stammzelle, wobei das genannte Verfahren den Schritt der Kultivierung der Zelle in dem Medium gemäß einem der Ansprüche 1 bis 3 umfasst.

5. Verfahren gemäß Anspruch 4, wobei die genannte epitheliale Stammzelle eine korneale epitheliale Stammzelle, eine konjunktivale epitheliale Stammzelle, eine epitheliale Stammzelle der Mundschleimhaut oder eine epitheliale Stammzelle der Haut ist.

## Revendications

1. Milieu de culture de cellules souches constitué
(a) d'un milieu basal sans sérum pour une culture de cellules animales choisi dans le groupe constitué de DMEM, F12 de Ham, DMEM/F-12 (1:1) ou RPMI1640 ;
(b) d'insuline, de transferrine, de sélénite de sodium, d'éthanolamine, de sérum albumine, d'acide linoléique, d'acide linolénique, de rétinol et d'α-tocophérol,
(c) et facultativement d'une ou de plusieurs choisies dans le groupe constitué de la SOD, la catalase, la glutathion-peroxydase, la triiodothyronine, la putrescine, la progestérone,
(d) et facultativement en outre d'un ou de plusieurs choisis dans le groupe constitué de EGF, FGF-7, et FGF-2 ;
lequel milieu ne contient pas de sérum sanguin.

2. Milieu selon la revendication 1, dans lequel ladite sérum albumine est de la sérum albumine humaine.

3. Milieu selon la revendication 2, dans lequel ladite sérum albumine humaine est une sérum albumine humaine recombinante.

4. Procédé de culture d'une cellule souche épithéliale, ledit procédé comprenant l'étape de culture de la cellule dans le milieu selon l'une quelconque des revendications 1 à 3.

5. Procédé selon la revendication 4, dans lequel ladite cellule souche épithéliale est une cellule souche épithéliale cornéenne, une cellule souche épithéliale conjonctivale, une cellule souche épithéliale de la muqueuse buccale, ou une cellule souche épithéliale de la peau.


### SEQUENCE LISTING

<110> Mitsubishi Tanabe Pharma Corporation
<120> Method for isolating cells, serum-free cell culture medium, and method
   for culturing cells
<130> A07023-C7315
<150> JP2007-184897
   <151> 2007-07-13
<160> 2
<170> PatentIn version 3.3
<210> 1
   <211> 21
   <212> DNA
   <213> Artificial sequence
<220>
   <223> primer
<400> 1
   tccaccatat accgccacag a 21
<210> 2
   <211> 23
   <212> DNA
   <213> Artificial sequence
<220>
   <223> primer
<400> 2
   atggtgccgt tgtaatttgt tgt 23